# EUROPEAN PATENT SPECIFICATION

(11) **EP 3 814 777 B1**
(45) Date of publication and mention of the grant of the patent: **05.11.2025**
(21) Application number: 19765338.9
(22) Date of filing: 28.06.2019
(51) Int. Cl.: G01N 33/569, G01N 33/92

(54) **METHOD FOR DIAGNOSIS OF LYME ARTHRITIS, METHOD FOR DIFFERENTIAL DIAGNOSIS OF LYME ARTHRITIS, LYSOPHOSPHATIDYLETHANOLAMINE FOR USE AS BIOMARKER, KIT FOR DIAGNOSIS OF LYME ARTHRITIS AND KIT FOR DIFFERENTIAL DIAGNOSIS OF LYME ARTHRITIS**
VERFAHREN ZUR DIAGNOSE VON LYME-ARTHRITIS, VERFAHREN ZUR DIFFERENTIALDIAGNOSE VON LYME-ARTHRITIS, LYSOPHOSPHATIDYLETHANOLAMIN ZUR VERWENDUNG ALS BIOMARKER, KIT ZUR DIAGNOSE VON LYME-ARTHRITIS UND KIT ZUR DIFFERENTIALDIAGNOSE VON LYME-ARTHRITIS
MÉTHODE DE DIAGNOSTIC DE L'ARTHRITE DE LYME, MÉTHODE DE DIAGNOSTIC DIFFÉRENTIEL DE L'ARTHRITE DE LYME, LYSOPHOSPHATIDYLÉTHANOLAMINE DESTINÉ À ÊTRE UTILISÉ EN TANT QUE BIOMARQUEUR, KIT DE DIAGNOSTIC DE L'ARTHRITE DE LYME ET KIT DE DIAGNOSTIC DIFFÉRENTIEL DE L'ARTHRITE DE LYME

(30) Priority: 29.06.2018 PL 42613818
(43) Date of publication of application: 05.05.2021
(73) Proprietor: Uniwersytet Medyczny W Bialymstoku, 15-089 Bialystok (PL)
(72) Inventor: LUCZAJ, Wojciech, 15-683 Bialystok (PL); MONIUSZKO-MALINOWSKA, Anna, 15-864 Bialystok (PL); DOMINGUES, Pedro Miguel Dimas Neves, 3810-416 Aveiro (PT); DOMINGES, Maria do Rosário Gonçalves dos Reis Marques, 3810-416 Aveiro (PT); GINDZIENSKA-SIESKIEWICZ, Ewa, 15-196 Bialystok (PL); SKRZYDLEWSKA, Elzbieta, 15-066 Bialystok (PL)
(74) Representative: Kawczynska, Marta Joanna
(86) International application number: PCT/PL2019/000047
(87) International publication number: WO 2020/005085

(56) References cited:
- US-A1- 2015 192 597
- MONIUSZKO-MALINOWSKA ANNA ET AL: "Lipid peroxidation in the pathogenesis of neuroborreliosis", FREE RADICAL BIOLOGY & MEDICINE, ELSEVIER INC, US, vol. 96, 29 April 2016 (2016-04-29), pages 255 - 263, XP029599545, ISSN: 0891-5849, DOI: 10.1016/J.FREERADBIOMED.2016.04.032
- LUCZAJ W ET AL: "Phospholipidomic Analysis Reveals Changes in Sphingomyelin and Lysophosphatidylcholine Profiles in Plasma from Patients with Neuroborreliosis", LIPIDS, SPRINGER, DE, vol. 52, no. 1, 10 November 2016 (2016-11-10), pages 93 - 98, XP036130392, ISSN: 0024-4201, [retrieved on 20161110], DOI: 10.1007/S11745-016-4212-3
- TAM VINCENT C ED - LAMBRIS DR JOHN D: "Lipidomic profiling of bioactive lipids by mass spectrometry during microbial infections", SEMINARS IN IMMUNOLOGY, vol. 25, no. 3, 2013, pages 240 - 248, XP028776088, ISSN: 1044-5323, DOI: 10.1016/J.SMIM.2013.08.006
- VICTORIA A. BLAHO ET AL: "Lipidomic Analysis of Dynamic Eicosanoid Responses during the Induction and Resolution of Lyme Arthritis", JOURNAL OF BIOLOGICAL CHEMISTRY, vol. 284, no. 32, 1 June 2009 (2009-06-01), pages 21599 - 21612, XP055634213, ISSN: 0021-9258, DOI: 10.1074/jbc.M109.003822

## Description

The subject matter of the invention relates to a method for *in vitro* diagnosis of Lyme arthritis, a method for *in vitro* differential diagnosis of Lyme arthritis versus rheumatoid arthritis, lysophosphatidylethanolamine comprising myristic acid for use as a biomarker of Lyme arthritis, and also as a biomarker for differential diagnosis of (tick-borne) Lyme arthritis.

### Technical Field

The present invention relates generally to the field of medicine, more specifically to the diagnostics of Lyme disease, especially Lyme arthritis, and in particular to differential diagnosis of Lyme arthritis versus reumathoid arthritis, with the use of a specific biological marker, i.e. a lysophospholipid comprising myristic acid, as a biomarker of Lyme disease, and in particular a biomarker of Lyme arthritis, and also as a biomarker for differential diagnosis of (tick-borne) Lyme arthritis. A kit for *in vitro* diagnosis of Lyme arthritis and a kit for *in vitro* differential diagnosis of Lyme arthritis have also been herein described.

### Background Art

Lyme disease, also known as Lyme borreliosis or a disease caused by Lyme disease spirochetes, is an infectious disease which results from an infection caused by the bacterium *Borrelia burgdorferi* transmitted to an organism by the bite of an infected tick. Lyme disease occurs in human subjects, in particular from regions endemic for Lyme disease, or persons exposed to the risk of getting the disease, for example foresters and woodcutters.

In recent years, a significant increase in the incidence of Lyme disease in human subjects has been observed. Presently, the disease has a global spread and thus 250,000 cases of the disease are reported yearly in the USA, whereas in Europe the incidence is 200,000 cases, of which 15,000 cases of the disease are reported in Poland. In a human subject the disease is caused by several species of Lyme disease spirochete, presently described as the burgdorferi group or *Borrelia burgdorferi* sensu lato, which comprises *Borrelia burgdorferi* sensu stricto, *B. garinii* and *B. afzelii.*

The problem of Lyme disease infections concerns also pets and domestic animals, which may contribute to significant economic losses in those sectors.

Lyme disease develops through three distinct stages, from an early stage of the infection until a late stage. In the early stage (Stage 1) the disease may be asymptomatic or may have flu-like symptoms. In 50-80% of cases a skin inflammatory rash can be observed several days after the tick bite, which has a very characteristic appearance and is called *erythema migrans* (EM). If left untreated, the disease gives various pathological symptoms, including articular, neurological, dermatological and heart symptoms. After several weeks from the infection, the disease transforms into Stage 2, which is manifested, among others, in Lyme arthritis (LA) and neurological disorders (neuroborreliosis). After several months or years from the infection, the disease evolves into a chronic atrophic form, the so-called Stage 3, which may involve the development of e.g. encephalopathy and/or encephalomyelitis. Similarity between the symptoms of Lyme disease and those of other unrelated diseases, as well as variability of those symptoms, make the clinical diagnosis difficult. The early stage of the disease may be asymptomatic until the disease develops into very advanced clinical stages, such as (tick-borne) Lyme arthritis or neuroborreliosis. In late stages of the disease additional, other, non-specific symptoms may appear which are difficult to diagnose using presently available diagnostic means.

Presently, the diagnosis of Lyme disease is based on immunological tests detecting the presence of *anti-Borrelia burgdorferi* anitbodies in blood. These tests are usually performed by ELISA technique and then confirmed by Western blott technique. However, *Borrelia burgdorferi* sensu lato expresses various surface proteins by adaptation to various environments, which results in the genetic diversity and varied expression of *Borrelia burgdorferi* genes. This situation has a significant impact on the possibility of developing specific and sensitive kits for the diagnosis of Lyme disease. *Anti-Borrelia burgdorferi* IgM antibodies usually appear within several days or weeks from the beginning of the infection and may remain in the course of the disease. IgG antibodies appear later, in the majority of patients - about one month from the beginning of active infection, and they may remain in the organism for many years from the infection and elimination of symptoms. The assessment of serological response is complicated considering the multitude of species causing the disease and due to cross-species diversity for the main immunodominant antigens, as well as genetic diversity. The results of serological tests with those antigens in terms of specificity and sensitivity are highly variable. Therefore, such tests are characterized by insufficient specificity due to possible cross-reactivity with antibodies connected with different pathogens, such as *Treponema pallidum,* other spirochetes, *Rickettsia* or *Helicobacter pylori.* For this reason, it is necessary to carry out additional Western blot analyses in the case of positive samples in immunological tests in order to confirm the initial results obtained by ELISA technique. It is actually considered that an elevated level of antibodies in diseased persons is an insufficient diagnostic factor; therefore there is a need to develop an unambigius biomarker of Lyme disease, including Lyme arthritis and neuroborreliosis. Moreover, there are still reported cases of the disease, despite negative results of tests for antibodies.

The diagnosis of Lyme arthritis is additionally made more complicated by the fact that there is a disease which has practically the same clinical symptoms, i.e. rheumatoid arthritis (RA). Both diseases, despite almost identical symptoms involving *inter alia* joint pains, reuquire a totally different treatment, allowing for a prognosis of a positive outcome thereof. Therefore, there is a need for specific and sensitive means for differential diagnosis of Lyme arthritis versus rheumatoid arthritis, which will make it possible to apply adequate treatment and cure the disease.

Similarly, the diagnosis of neuroborreliosis is made more complicated by the fact that there is another disease entity which has very similar symptoms, i.e. tick-borne encephalitis (TBE). Unlike neuroborreliosis, tick-borne encephalitis is caused by a virus and not bacterium. Thus, both diseases, despite almost identical symptoms, require a totally different treatment. Therefore, there is a need for specific means and diagnostic methods allowing unambiguous clinical diagnosis of those diseases, which will make it possible to apply adequate treatment and curing of the disease.

US 2015/192597 discloses 14:0-lysoPE as a biomarker, but does not disclose lysophosphatidylethanolamine comprising myristic acid as a biomarker in the *in vitro* diagnosis of Lyme arthritis and differential diagnosis of Lyme arthritis versus rheumatoid arthritis.

The publication by Luczaj W et al., Phospholipidomic analysis reveals changes in sphingomyelin and lysophosphatidylcholine profiles in plasma from patients with neuroborreliosis, LIPIDS, SPRINGER, DE, vol. 52, no. 1, 10 November 2016, pp. 93-98, discloses use of different phospholipids (PLs) for the diagnosis of neuroborreliosis, but does not disclose lysophosphatidylethanolamine comprising myristic acid as a biomarker in the *in vitro* diagnosis of Lyme arthritis and differential diagnosis of Lyme arthritis versus rheumatoid arthritis.

Presently available diagnostic means and methods are therefore insufficient. There is therefore a need for specific and sensitive methods for *in vitro* diagnosis of Lyme disease, in particular Lyme arthritis, and especially *in vitro* differential diagnosis of Lyme arthritis versus rheumatoid arthritis. There is also an urgent need for providing specific means, including biomarkers, for specific diagnosis of Lyme disease, especially Lyme arthritis, in particular for diffferential diagnosis versus other disease entities.

The object of the invention is therefore to provide methods and means for specific and sensitive diagnosis of Lyme arthritis, and differential diagnosis of Lyme arthritis versus rheumatoid arthritis, , such as specific and sensitive biomarkers, as well as diagnostic methods using such biomarkers.

### Summary of Invention

The above objects have been achieved by the solutions claimed in the attached patent claims. Preferable variants of the invention are defined in the dependent claims.

The subject matter of the invention relates to a method for *in vitro* diagnosis of Lyme arthritis in a subject, characterized in that:
a) in a sample from a subject, the level of lysophosphatidylethanolamine comprising myristic acid (LysoPE(14:0)) is determined, and
b) the level of lysophosphatidylethanolamine determined in a) is compared with the level of lysophosphatidylethanolamine comprising myristic acid in a reference sample;

wherein the reference sample is a sample from a healthy control subject;
wherein the sample is: whole blood, plasma or serum; and
wherein the level of lysophosphatidylethanolamine comprising myristic acid which is higher than the level in the said reference sample indicates that the subject suffers from Lyme disease.

Preferably, in the method for *in vitro* diagnosis of Lyme arthritis according to the invention the sample from the subject is blood plasma.

Preferably, in the method for *in vitro* diagnosis of Lyme arthritis according to the invention the subject in a human subject.

Preferably, in the method for *in vitro* diagnosis of Lyme arthritis according to the invention the level of lysophosphatidylethanolamine comprising myristic acid is determined by liquid chromatography coupled with mass spectrometry (LC-MS) method, in particular LC-MS/MS method.

The subject matter of the invention further relates to a method for *in vitro* differential diagnosis of Lyme arthritis versus rheumatoid arthritis, characterized in that:
a) in a sample from a subject the level of lysophosphatidylethanolamine comprising myristic acid (LysoPE(14:0)) is determined, and
b) the level of lysophosphatidylethanolamine determined in a) is compared with the level in a reference sample;

wherein the reference sample is a sample from a healthy control subject;
wherein the sample is: whole blood, plasma or serum; and
wherein the level of lysophosphatidylethanolamine comprising myristic acid which is higher than the level in the said reference sample indicates that the subject suffers from Lyme arthritis.

Preferably, in the method for *in vitro* differential diagnosis according to the invention a sample selected from a group comprising whole blood, plasma and serum is used as the sample from a subject.

More preferably, in the method for *in vitro* differential diagnosis according to the invention the sample from the subject is blood plasma.

Preferably, in the method for *in vitro* differential diagnosis according to the invention the subject is a human subject.

Preferably, in the method for *in vitro* differential diagnosis according to the invention the level of lysophosphatidylethanolamine comprising myristic acid is measured by liquid chromatography coupled with mass spectrometry (LC-MS) method, in particular LC-MS/MS method.

The subject matter of the invention also relates to lysophosphatidylethanolamine comprising myristic acid (LysoPE(14:0)) for use as a biomarker of Lyme arthritis.

The subject matter of the invention also relates to lysophosphatidylethanolamine comprising myristic acid (LysoPE(14:0)) for use as a biomarker for differential diagnosis of Lyme arthritis versus rheumatoid arthritis.

### Detailed Description of Invention

A biomarker, in other words a biological marker or a bioindicator, is a biological indicator which enables qualitative and/or quantitative assessment of various medical, pathological and disease conditions and/or biological phenomena or features. In modern medicine the biomarkers play invaluable role; they enable, among others, a quick, precise, specific and sensitive diagnosis of various diseases and disorders. Such biomarkers are defined as molecular, genetic and biochemical factors used for a precise and easy diagnosis of diseases, for example, chronic, genetic diseases, cancers, as well as for the assessment of progression of such diseases or monitoring the treatment thereof, or the assessment of the probability of occurrence of such kind of diseases in the examined subject.

A significant development in the field of research concerning changes in lipid metabolism has been seen over last decade. That is why an analysis of phospholipid profile has been suggested for the purose of early clinical diagosis of many illnesses, e.g. for the purpose of characterizing neoplasms or other diseases - see e.g. publications by Wymann M. P., and Schneiter R. 2008. Lipid signalling in disease. Nat. Rev. Mol. Cell Biol. 9: 162-176 and Fernandis A. Z., and Wenk M. R. 2009. Lipid-based biomarkers for cancer. J. Chromatogr. B Analyt. Technol. Biomed. Life Sci. 877: 2830-2835. However, the analysis of phospholipids' profile has not been used so far for the diagnosis of Lyme disease, in particular Lyme arthritis.

The present inventors have identified and developed a new biomarker which enables specific and sensitive diagnosis of Lyme arthritis, as well as differential diagnosis of Lyme arthritis versus other diseases, i.e. rheumatoid arthritis (RA) . It has also been herein described that such biomarker may be used in the diagnosis of Lyme disease, including differential diagnosis of Lyme arthritis versus neuroborreliosis and tick-borne encephalitis.

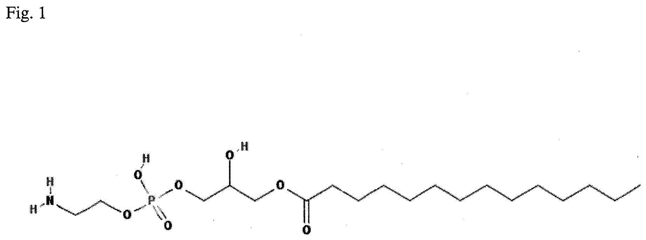

The biomarker identified by the present inventors is lysophosphatidylethanolamine comprising myristic acid (LPE (14:0) or 14:0 Lyso PE). It is a chemical compund having the molecual weight of 425.497 g/mol; the monoisotopic mass of 425.254 g/mol; the molecular formula: C₁₉H₄₀NO₇P and the structural formula as presented in Figure 1.

The subject matter of the invention thus relates to lysophosphatidylethanolamine comprising myristic acid (LysoPE(14:0)) for use as a biomarker of Lyme arthritis, and lysophosphatidylethanolamine comprising myristic acid (LysoPE(14:0)) for use as a biomarker for differential diagnosis of Lyme arthritis versus rheumatoid arthritis.

Phosphatidylethanolamine comprising myristic acid is an exogenous compound that is synthesized in bacterial, fungal and plant organisms, but this compound is not synthesized in animal organisms, including human subjects. Therefore, the presence of trace amounts of compounds comprising this acid, including phospholipids, especially phosphatidylethanolamine, in animal organisms, including human subjects, is due to an exogenous factor, e.g. a diet including high amounts of fats of plant origin. Phospholipids constitute a basic structural element of each cell membrane. Among all identified phospholipids isolated from *Borrelia burgdorferi* cells, a significant proportion of lysophospholipids comprising a specific acid identified, that is myristic acid, has been reported. Lysophospholipids (LPLs) belong to a group phospholipids that are formed as a result of hydrolisys of phospholipids (PLs) with the participation of phospholipase A2 (PLA2) enzyme. It has been found that the said enzyme plays a key role in the immunological response of an organism to a bacterial infection. An increased PLA2 activity in the plasma of patients with Lyme arthritis has also been confirmed. Additionally, it has been demonstrated that bacteria have a specific protein that transports LPLs within their cell membrane, as a result of which they accumulate on the inner part of the membrane. See e.g. the following publications: Belisle, J. T., Brandt, M. E., Radolf, J. D., & Norgard, M. V. (1994). Fatty acids of Treponema pallidum and Borrelia burgdorferi lipoproteins. Journal of bacteriology, 176(8), 2151-2157; Ben-Menachem, G., Kubler-Kielb, J., Coxon, B., Yergey, A., & Schneerson, R. (2003). A newly discovered cholesteryl galactoside from Borrelia burgdorferi. Proceedings of the National Academy of Sciences, 100(13), 7913-7918; Fraser, C. M., Casjens, S., Huang, W. M., Sutton, G. G., Clayton, R., Lathigra, R., & Gwinn, M. (1997). Genomic sequence of a Lyme disease spirochaete, Borrelia burgdorferi. Nature, 390(6660), 580-586.

However, so far lysophosphatidylethanolamine comprising myristic acid has neither been associated with the occurence of any disease entity nor it has been used a biomarker for the diagnosis of any disease.

As a result of studies carried out to assess changes in the phospholipid profile (unpublished data), the present inventors unexpectedly-found more than ten times higher concentration of lysophosphatidylethanolamine comrpising myristic acid in body fluids, such as plasma, from patients with Lyme disease, more specifically Lyme arthritis, as compared to healthy persons as well as patients with RA but not affected by Lyme disease, Lyme arthritis or neuroborreliosis. The present inventors found the presence of lysophosphatidylethanolamine comprising myristic acid in the plasma of patients with rheumatoic arthritis (RA); however the content of that compound was not statistically different from the content in healty persons.

Thus, the present invention provides lysophosphatidylethanolamine comprising myristic acid for use as a biomarker of Lyme arthritis, and in particular for differntial diagnosis of Lyme arthritis. The present invention also provides suitable *in vitro* diagnostic methods consisting in the determination of the said biomarker in a sample from a subjcet wherein an increased level or concentration of lysophosphatidylethanolamine comprising myristic acid in such a sample indicates the presence of Lyme arthritis, in the examined subject. The examined subject is preferably a human subject, but it may also be an animal which needs a diagnosis in respect of Lyme arthritis. The reference sample according to the invention is a sample obtained from a control subject, which means a healthy subject without Lyme disease, without neuroborreliosis, without RA and without TBE. A sample from a subject with RA or TBE, but who simultaeously does no have Lyme disease and Lyme arthritis and neuroborreliosis, may also constitute such a reference sample.

The present invention also provides a biomarker, and a method for differential diagnosis of Lyme arthritis versus rheumatoid arthritis (RA.

The present inventors have found a highly specific diagnostic relationship between lysophosphatidylethanolamine comprising myristic acid and Lyme arthritis. So far, lysophosphatidylethanolamine comprising myristic has not been used as a diagnostic biomarker and has not been associated with any disease entity, and it has not been used for diagnostic purposes either. The present inventors have demonstrated that lysophosphatidylethanolamine comprising myristic acid is a specific and sensitive biomarker of Lyme arthritis.

Determination of the level of lysophosphatidylethanolamine comprising myristic acid in the methods according to the invention may be conducted using standard methods known in this field. Preferably, the level thereof is determined using a highly-specialized analytical technique i.e. liquid chromatography coupled with mass spectrometry (LC-MS), or more preferably by LC-MS/MS method, which methods as such are known in this field. It is also possible to determine the level of this compound by other analytical methods known in this field. For this purpose, antiphospholipid antibodies (APa), for example, can be used, which are directed against phospholipids and phospholipid-binding plasma proteins. Such an analytical method simplifies the procedure for determining the level of lysophosphatidylethanolamine comprising myristic acid in a sample from a subject and at the same time is characterized by a high specificity of determination.

A diagnostic kit for the diagnosis of Lyme disease comprising a means for determining lysophosphatidylethanolamine comprising myristic acid has been herein described. A diagnostic kit for differential diagnosis of Lyme arthritis comprising a means for determining lysophosphatidylethanolamine comprising myristic acid has also been herein described. Such kits may be used for a precise diagnosis of Lyme disease and Lyme arthritis in subjects exhibiting initial symptoms of the disease confirmed by presently used immunologic methods. Such diagnostic kits enable a simple and quick detection of the presence of lysophosphatidylethanolamine comprising myristic acid and its quantitative assessment in the examined sample from a subject, e.g. in plasma, and thus such kits are useful diagnostic tools which may allow one to make a diagnosis of Lyme disease, and especially differential diagnosis of Lyme arthritis versus RA. Such means for determining lysophosphatidylethanolamine comprising myristic acid in the kits herein described may be any means suitable for determining lysophosphatidylethanolamine comprising myristic acid in a sample from a subject, such as a sample of the body fluid from a subject, for example, cerebrospinal fluid or preferably plasma. For example, these may be antiphospholipid antibodies binding to this biomarker, i.e. LPE (14:0).

As a result of studies carried out by the present inventors to assess changes in the phospholipid profile in persons with Lyme arthritis versus healthy persons (unpublished data) and versus persons suffering from RA, it was unexpectedly found that the concentration of the lysophospholipid comprising specific acid, i.e. lysophosphatidylethanolamine comprising myristic acid, in the plasma of patients with Lyme arthritis was more than ten times higher compared to healthy persons. It was thus demonstrated that the presence of lysophosphatidylethanolamine comprising myristic acid in body fluids, such as blood plasma, is a useful biomarker of Lyme disease, especially Lime arthritis..

Lysophosphatidylethanolamine comprising myristic acid is present only in trace amounts in the plasma of healthy human subjects, which fact also applies to other phospholipids comprising myristic acid. This is also true for patients with RA. It results from the fact that myristic acid in the identified lysopholipid is an exogenous compound synthesized in bacterial, fungal or plant organisms, whereas it is not synthesized in the animal organism, inluding human organism. Therefore, the presence of trace amounts of compunds comprising myristic acid, including phospolipids, in the human organism is caused by a diet including large amounts of fats of plant origin. Phospholipids constitue a basic structural element of every cell membrane. Among all identified phospholipids isolated from *Borrelia burgdorferi* cells, a significant proportion of phospholipids comprising the identified specific acid has been reported. Lysophospholipids (LPLs) belong to a group of phospholipids formed as a result of hydrolisys of phospholipids (PLs) with the participation of phospholipase A2 (PLA2) enzyme. It has been found that the said enzyme plays a key role in the immunological response of an organism to a bacterial infection. Increased activity of PLA2 in the plasma of patients with Lyme arthritis has been also confirmed. Additionally, it has been demonstrated that bacteria have a specific protein that transports LPLs within their cell membrane, as a result of which they accumulate on the inner part of the membrane.

All the above information, when taken together, provides a basis for the conclusion that an increased content of the lysophospholipid comprising myristic acid in the samples from patients with Lyme disease, in particular with Lyme arthritis, results from the presence of *Borrelia burgdorferi* in the subject's organism, which fact simultaneously indicates that the examined subject suffers from Lyme disease or Lyme arthirtis, respectively, and confirms the usefulness of this compound as a biomarker of these diseases as well as its usefulness in the methods for *in vitro* diagnosis of these diseases.

Additionally, in the case of Lyme arthritis, the diagnosis of which is additionally made more complicated by the presence of a disease which has practically identical clinical symptoms, i.e. rheumatoid arthritis (RA), the invention enables differential diagnosis of Lyme arthritis versus rheumatoid arthritis. Both diseases, despite almost identical symptoms, require a totally different treatment. As a result of studies carried out by the present inventors to assess changes in the phospholipid profile in persons with rheumatoid arthritis versus healthy persons (unpublished data), the presence of lysophospholipid comprising myristic acid was reported in the plasma of patients with RA; however, the content of that compund was not statistically different from the content thereof in the healthy persons. Therefore, the present invention provides lysophosphatidylethanolamine comprising myristic acid (LysoPE(14:0)) as a biomarker of Lyme arthritis, and it further provides a method for *in vitro* diagosis of this disease, which makes it possible to avoid diagnostic errors indicating RA. Preferably, the body fluid used in the diagnostic method according to the invention is plasma.

The present invention will now be illustrated in the example below. Unless indicated otherwise, all methods and parameters are as commonly used in the field to which this invention belongs, and the applied devices and reagents are used in a manner as recommended by the manufacturers thereof.

### Description of Figures

**Figure 1** - shows the structural formula of lysophosphatidylethanolamine comprising myristic acid.

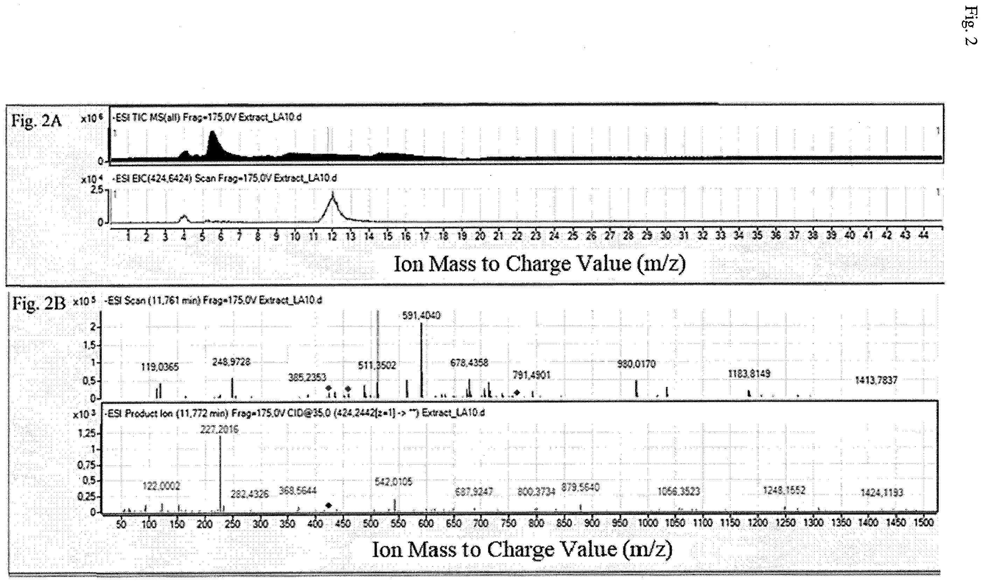

**Figure 2** - Fig. 2A shows a representative Total Ion Chromatogram (TIC) in negative ionisation mode for blood plasma of a patient with rheumatoid arthritis (RA) (the top chromatogram); Extracted Ion Chromatogram (EIC) for m/z 424.6424 in negative ionisation mode (the bottom chromatogram); Fig. 2B - shows a representative mass spectrum MS of the peak at RT = 12.06 min (the top spectrum); the fragmentation mass spectrum MS/MS of the ion having m/z 424.2442 corresponding to the molecular ion of LPE (14:0) (the bottom spectrum) - the m/z 227.2016 signal confirms the presence of myristic acid (14:0) in the structure of the fragmented compound.

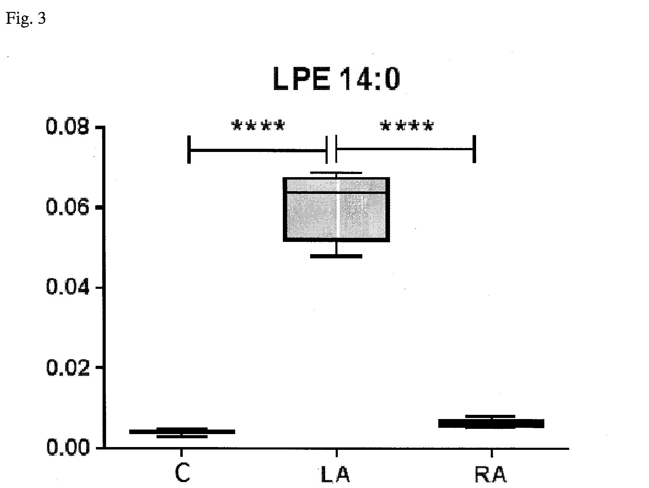

**Figure 3** - shows a box-and-whisker plot which presents graphically changes in the relative content of LPE (14:0) in healthy persons, patients with rheumatoid arthritis and patients with Lyme arthritis. (p<0.0001****). LA - Lyme arthritis; RA - rheumatoid arthritis; C - control.

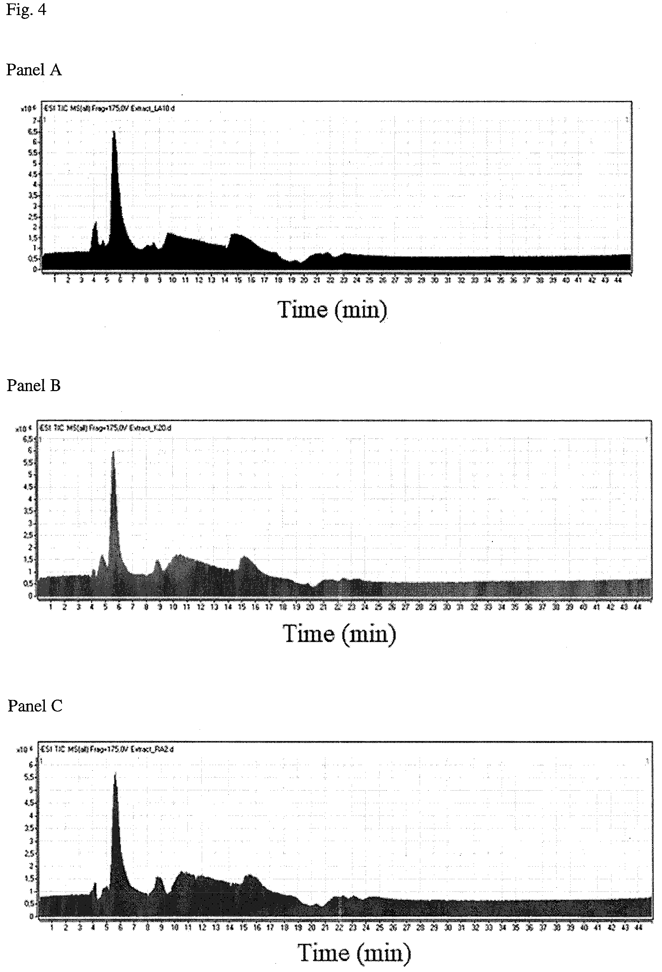

**Figure 4** - shows representative Total Ion Chromatograms (TICs), as a phospholipid profile in negative ionization mode: plasma from a patient with Lyme arthritis - panel A; plasma from a healthy person - panel B; plasma from a patient with rheumatoid arthritis - panel C.

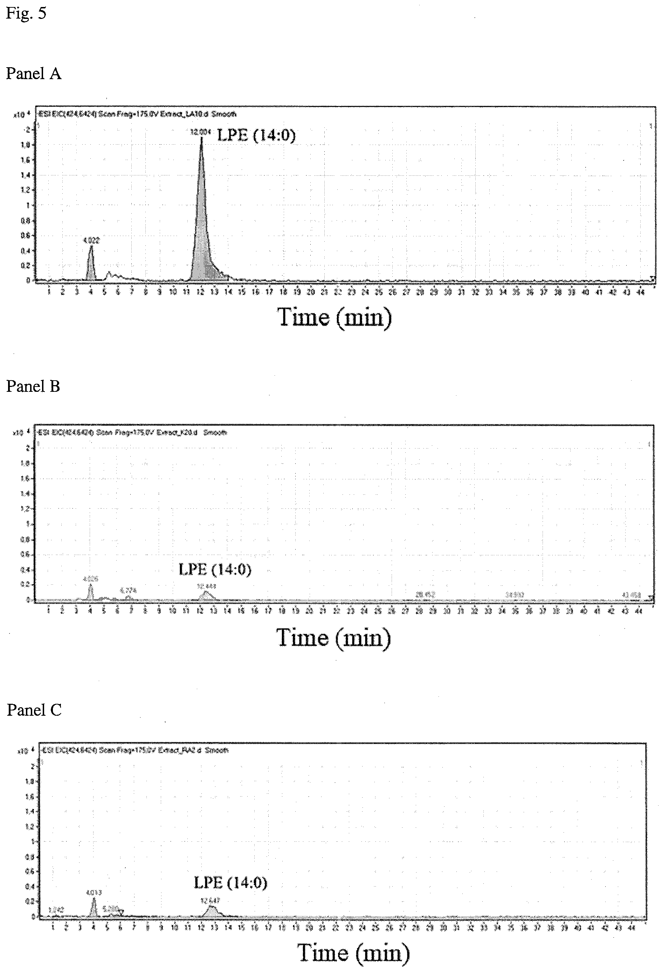

**Figure 5** - shows representative Extracted Ion Chromatograms (EICs) in negative ionization mode for m/z 424.6424 corresponding to the molecular ion of LPE(14:0), retention time RT = 12 min: plasma from a patient with Lyme arthritis - panel A; plasma from a healthy person - panel B; plasma from a patient with rheumatoid arthritis - panel C.

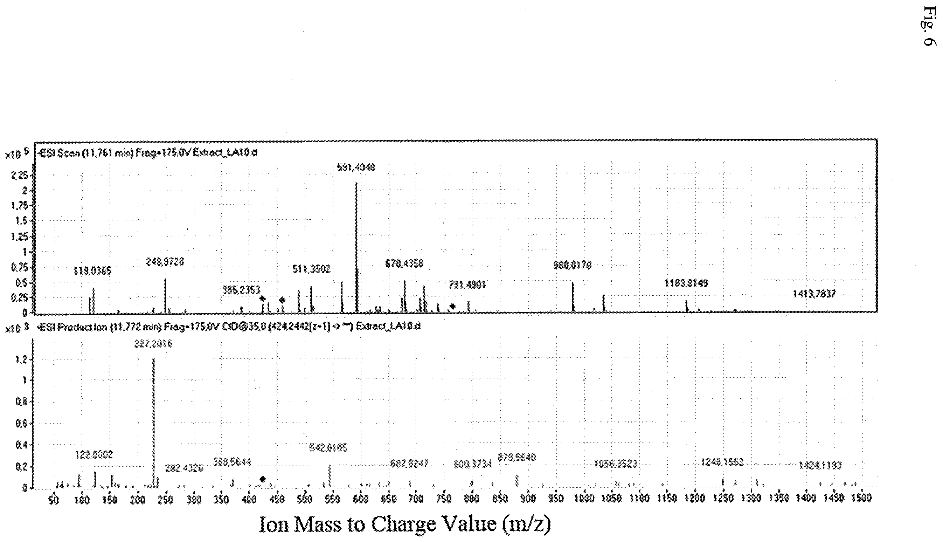

**Figure 6** - shows mass spectrum MS of the peak at RT=12.06 min (the top panel); the fragmentation mass spectrum MS/MS of the ion having m/z 424.2442 corresponding to the molecular ion of LPE (14:0) (the bottom panel) - the m/z 227.2016 signal confirms the presence of myristic acid (14:0) in the structure of the fragmented compound.

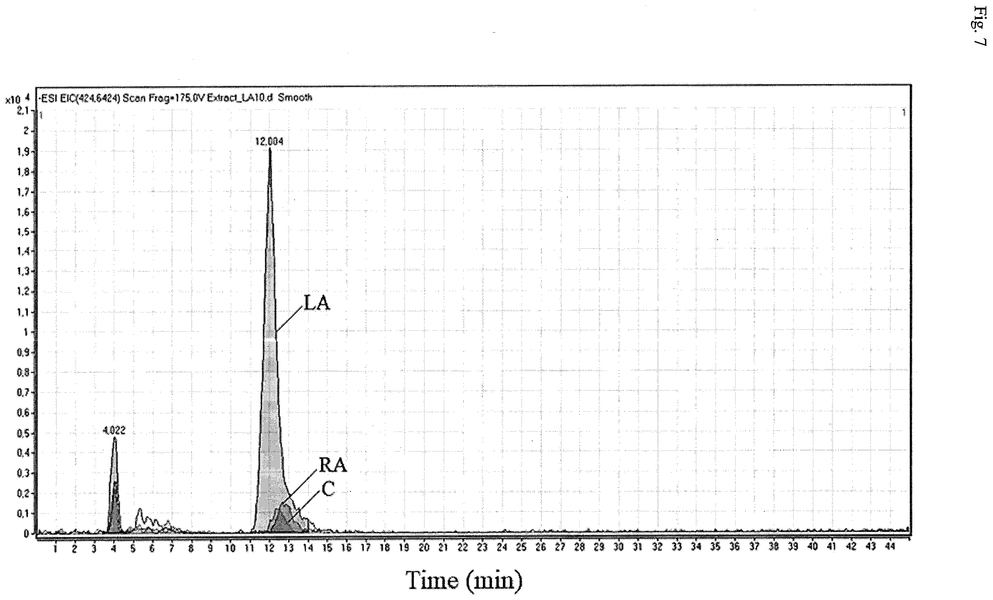

**Figure 7** - shows representative overlaid-mode Extracted Ion Chromatograms (EICs) in negative ionization mode for m/z 424.6424 corresponding to the molecular ion of LPE(14:0), retention time RT = 12 min: LA - plasma from a patient with Lyme arthritis; C - plasma from a healthy person; RA - plasma from a patient with rheumatoid arthritis.

### Example - Determination of the LPE (14:0) level

### Test material

The biological material used for the analyses was plasma obtained from the venous blood of persons suffering from rheumatoid arthritis (RA), persons suffering from Lyme arthritis (LA) and healthy persons. LA group consisted of 9 patients with diagnosed (tick-borne) Lyme arthritis (2 women and 7 men) aged from 22 to 81 years (the mean age was 49 years) hospitalized in the Department of Infectious Diseases and Neuroinfection of the University of Bialystok. The illness was diagnosed on the basis of the presence of anti-*Borrelia burgdorferi* specific IgM and/or IgG antibodies in blood serum, detected by ELISA method. RA group consisted of 9 patients with active RA (2 women and 7 men) aged 23 to 79 years (the mean age was 48 years). The assessment of activity of the disease was based on the four-variable DAS28-CRP parameter. The control group consisted of 9 healthy persons (2 women and 7 men) aged from 24 to 71 years (the mean age was 47 years). Venous blood was obtained from both the patients and healthy persons to heparinized test tubes. The samples were centrifuged at 2000 x g, in 4°C for 20 minutes in order to obtain plasma. After adding buthylhydroxytoluene (BHT), acting as an antioxidant, the samples were kept at - 80°C until analysis. The study was started after obtaining the consent of the Bioethics Committee of the Medical University of Bialystok and a written consent of each person participating in the study.

The compound was identified in both the plasma from patients and the plasma from healthy persons by a highly specialized analytical technique LC-MS using hydrophilic interaction liquid chromatography (HILIC), after previous isolation of lipid fraction by the solvent-solvent extraction method as described in detail below.

### Extraction of lipids from blood plasma

Extraction was carried out in glass test tubes previously washed with chloroform (CHCl₃) in order to remove any impurities. 200 µl of plasma and 1.5 ml of methanol (MeOH) cooled to -20°C was placed in each test tube. The mixture obtained was vortexed for 10 minutes. Then, 3 ml of CHCl₃ cooled to -20°C was added and was again vortexed for 3 minutes. In the next stage, extracted samples were incubated in ice for one hour. During incubation, from time to time, the content of the test tubes was vortexed for a moment. After incubation, in order to initiate separation of phases, 1.25 ml of ultrapure (Milli-Q) water was added and the samples were again left in ice for 10 minutes, their content being mixed in Vortex shaker from time to time. Then, the samples were centrifuged at 2500 x g for 10 minutes. After centrifuging, the bottom organic phase was transferred to a new glass test tube and another extraction was carried out by adding 2 ml of CHCl₃/MeOH (2:1, v/v) mixture cooled to -20°C. The next obtained portion of chloroform layer was combined with the previously collected portion and evaporated to dry residue in a nitrogen atmosphere. The extracts obtained were kept in -80°C until analysis.

### Determination of the profile of phospholipids in blood plasma using the LC-MS-QTOF system

To obtain the profile of phospholipids, the obtained extracts of blood plasma lipids from the patients with rheumatoid arthritis, patients with Lyme arthritis and healthy persons were analysed using LC-MS-QTOF in data dependent MS/MS mode (auto MS/MS). Separation of phospholipids classes was carried out with the use of hydrophilic interaction liquid chromatography (HILIC) using Ascentis Si HPLC Pore column, 15 cm x 1.0 mm, 3 mm; (Sigma-Aldrich) and gradient elution using a combination of two mobile phases A and B. Mobile phase A contained 25% water, 50% acetonitrile, and 25% v/v methanol and 10 mM addition of salt in the form of ammonium acetate. Mobile phase B contained 60% acetonitrile, 40% methanol and 10 mM addition of ammonium acetate. The gradient applied at 40 ml/min mobile phase flow used a mixture having the following composition:
0% mobile phase A and 100% mobile phase B [0 min]
100% mobile phase A and 0% mobile phase B [20 min]
100% mobile phase A and 0% mobile phase B [35 min]
0% mobile phase A and 100% mobile phase B [45 min]

Mobile phase B was used as a solvent for samples of extracts comprising 20 µg of phospholipids. 5 µl of such prepared samples were injected into the chromatographic column.

Samples of the extracts were analysed in negative ionisation mode using electrospray ionisation (ESI) sources.

Agilent Technologies ultra-performance liquid chromatograph (UPLC), series 1290; Agilent Technologies QTOF mass detector, 6540, equipped with an electrospray ionization (ESI) source; Peak Scientific LC-MS 20 nitrogen generator; Ascentis Si HPLC Pore chromatographic column, 15 cm x 1.0 mm, 3 mm, Sigma-Aldrich were used for the diagnosis by the method according to the invention. However, the methods according to the invention may be carried out using any other devices, systems and analytical kits which enable quantitative determination of lysophosphatidylethanolamine comprising myristic acid.

### Statistical analysis

The results obtained were subjected to statistical analysis in the following way. Fold change of relative LPE (14:0) content in healthy persons, patients with rheumatoid arthritis and patients with Lyme arthritis was analysed. The statistical analysis was performed based on one-way ANOVA analysis. Relative amount of LPE (14:0) was calculated by dividing the peak area of LPE (14:0) obtained on the basis of Extracted Ion Chromatograms (EICs) by the peak area of PC (28:0) being an Internal Standard (ISTD). The results were expressed as arithmetical means and mean standard errors. The comparisons between the control group and the persons suffering from rheumatoid arthritis and Lyme arthritis were made using one-way ANOVA analysis and the post hock test: Tukey's HSD test, with the use of the statistical package GraphPad Prism v.7.0, GraphPad Software, USA. The differences at p < 0.05 were considered as statistically significant.

The results obtained are presented in Table 1 below and in Figures 2 to 7.

**Table 1. Fold change of relative content of LPE (14:0) in healthy persons, patients with rheumatoid arthritis and patients with Lyme arthritis. Abbreviations: LA - Lyme arthritis; RA - rheumatoid arthritis; LPE - lysophosphatidylethanolamine; RT - retention time; ID - abbreviation of compound name; C - control.**

| **m/z** | **RT** | **ID** | **Log₂ (fold change)** | | | | | |
|---|---|---|---|---|---|---|---|---|
| | | | LA versus C | *Value p* | RA versus C | *Value p* | LA versus RA | *Value p* |
| 424.2464 | 12.38 | LPE (14:0) | 3.937 | <0.0001 | 0.691 | ns | -3.246 | <0.0001 |

Figure 3 shows a box-and-whisker plot which presents graphically changes in the relative content of LPE (14:0) in healthy persons (C), patients with rheumatoid arthritis (RA) and patients with Lyme arthritis (LA). (p<0.0001****). It has been demonstrated that the relative content of LPE (14:0) in patients with Lyme arthritis is statistically significantly higher compared to both healthy human subjects (control) and patients with RA.

Figure 4 shows representative exemplary Total Ion Chromatograms (TICs) as a phospholipid profile in negative ionisation mode: plasma from a patient with Lyme arthritis - panel A; plasma from a healthy person - panel B; plasma from a patient with rheumatoid arthritis - panel C.

Figure 5 shows representative exemplary Extracted Ion Chromatograms (EICs) in negative ionisation mode for m/z 424.6424 corresponding to the molecular ion of LPE (14:0), retention time RT=12 min: plasma for a patient with Lyme arthritis - panel A; plasma from a healthy person - panel B; plasma from a patient with rheumatoid arthritis - panel C. The difference in the obtained chromatogram profile of a patient with Lyme arthritis (panel A) can be clearly seen, which indicates the presence of LPE (14:0) and at the same time allows the diagnosis of the Lyme disease. A markedly higher peak of lysophospholipid comprising myristic acid, i.e. LPE (14:0) can be noticed in panel A as opposed to panels B and C.

Figure 6 shows MS mass spectrum of the peak at RT=12.06 min (top panel); fragmentation MS/MS mass spectrum of an ion having m/z 424.2442 corresponding the molecular ion of LPE (14:0) (bottom panel) - the m/z 227.2016 signal confirms the presence of myristic acid (14:0) in the structure of the fragmented compound.

Figure 7 shows representative overlaid mode Extracted Ion Chromatograms (EICs) in negative ionization mode for m/z 424.6424 corresponding to the molecular ion of LPE (14:0), retention time RT=12 min: plasma from a patient with Lyme arthritis - LA; plasma from a healthy person - C; plasma from a patient with rheumatoid arthritis - RA. It can be clearly seen that the highest peak was obtained for the patient with Lyme arthritis, which indicates that the method according to the invention using a biomarker in the form of lysophosphatidylethanolamine comprising myristic acid enables specific diagnosis of Lyme disease, in particular the diagnosis of Lyme arthritis versus a healthy person and differential diagnosis versus a person suffering from RA (but which person does not suffer from Lyme disease or Lyme arthritis or neuroborreliosis)

The data presented confirm that the methods according to to the invention enable precise, sensitive and specific differentiation between a subject suffering from Lyme arthritis, and both a healthy subject and a subject suffering from RA (but which subject does not suffer from Lyme disease or Lyme arthritis).

In summary, as it can be seen in the above Table 1 and in the presented Figures, the statistically significant differences in the content of LPE (14:0) were obtained in the studied samples from persons suffering from Lyme disease, especially Lyme arthritis versus the samples from healthy persons as well as persons suffering from rheumatoid arthritis (but without Lyme disease or Lyme arthritis or neuroborreliosis). In the samples of plasma obtained from patients with Lyme arthritis the relative content of LPE (14:0) was significantly higher than in the samples of plasma from healthy persons and persons with RA not suffering from Lyme disease/Lyme arthritis. The results obtained indicate that LPE (14:0) may be effectively used as a biomarker of Lyme disease, and in paraticular as a biomarker of Lyme arthritis, enabling specific diagnosis of Lyme arthritis, especially differential diagnosis of Lyme arthritis versus rheumatoid arthritis.

## Claims

1. A method for *in vitro* diagnosis of Lyme arthritis in a subject, **characterized in that**:
a) in a sample from a subject, the level of lysophosphatidylethanolamine comprising myristic acid (LysoPE(14:0)) is determined, and
b) the level of lysophosphatidylethanolamine determined in a) is compared with the level of lysophosphatidylethanolamine comprising myristic acid in a reference sample;
wherein the reference sample is a sample from a healthy control subject;
wherein the sample is: whole blood, plasma or serum; and
wherein the level of lysophosphatidylethanolamine comprising myristic acid which is higher than the level in the said reference sample indicates that the subject suffers from Lyme arthritis.

2. The method for *in vitro* diagnosis of Lyme arthritis according to claim 1, **characterized in that** the sample is blood plasma.

3. The method for *in vitro* diagnosis of Lyme arthritis according to claim 1 or 2, **characterized in that** the subject is a human subject.

4. The method for *in vitro* diagnosis of Lyme arthritis according to any one of claims 1 to 3, **characterized in that** the level of lysophosphatidylethanolamine comprising myristic acid is determined by liquid chromatography coupled with mass spectrometry (LC-MS) method, in particular LC-MS/MS method.

5. A method for *in vitro* differential diagnosis of Lyme arthritis versus rheumatoid arthritis, **characterized in that**:
a) in a sample from a subject, the level of lysophosphatidylethanolamine comprising myristic acid (LysoPE(14:0)) is determined, and
b) the level of lysophosphatidylethanolamine determined in a) is compared with the level in a reference sample;
wherein the reference sample is a sample from a healthy control subject;
wherein the sample is: whole blood, plasma or serum; and
wherein the level of lysophosphatidylethanolamine comprising myristic acid which is higher than the level in the said reference sample indicates that the subject suffers from Lyme arthritis.

6. The method for *in vitro* differential diagnosis according to claim 5, **characterized in that** the sample is blood plasma.

7. The method for *in vitro* differential diagnosis according to claim 5 or 6, **characterized in that** the subject is a human subject.

8. The method for *in vitro* differential diagnosis according to any one of claims 5 to 7, **characterized in that** the level of lysophosphatidylethanolamine comprising myristic acid is measured by liquid chromatography-mass spectrometry (LC-MS) method, in particular LC-MS/MS method.

9. Use of lysophosphatidylethanolamine comprising myristic acid (LysoPE(14:0)) as a biomarker in the *in vitro* diagnosis of Lyme arthritis.

10. Use of lysophosphatidylethanolamine comprising myristic acid (LysoPE(14:0)) as a biomarker in the *in vitro* differential diagnosis of Lyme arthritis versus rheumatoid arthritis.

## Patentansprüche

1. Verfahren zur *in-vitro-Diagnose* von Lyme-Arthritis bei einem Probanden, **dadurch gekennzeichnet, dass**:
a) in einer Probe eines Probanden der Gehalt an Lysophosphatidylethanolamin, das Myristinsäure enthält (LysoPE(14:0)), bestimmt wird, und
b) der in a) ermittelte Lysophosphatidylethanolamin-Gehalt mit dem Gehalt an Lysophosphatidylethanolamin, das Myristinsäure enthält, in einer Referenzprobe verglichen wird;
wobei die Referenzprobe eine Probe eines gesunden Kontrollprobanden ist;
wobei die Probe Vollblut, Plasma oder Serum ist; und
wobei ein höherer Gehalt an Lysophosphatidylethanolamin, das Myristinsäure enthält, als der Gehalt in der Referenzprobe darauf hinweist, dass der Proband an Lyme-Arthritis leidet.

2. Verfahren zur *in-vitro-Diagnose* von Lyme-Arthritis nach Anspruch 1, **dadurch gekennzeichnet, dass** die Probe Blutplasma ist.

3. Verfahren zur *in-vitro-Diagnose* von Lyme-Arthritis nach Anspruch 1 oder 2, **dadurch gekennzeichnet, dass** es sich bei dem Probanden um einen Menschen handelt.

4. Verfahren zur *in*-*vitro*-Diagnose von Lyme-Arthritis nach einem der Ansprüche 1 bis 3, **dadurch gekennzeichnet, dass** der Gehalt an Lysophosphatidylethanolamin, das Myristinsäure enthält, mittels Flüssigchromatographie in Verbindung mit Massenspektrometrie (LC-MS), insbesondere LC-MS/MS, bestimmt wird.

5. Verfahren zur *in*-*vitro*-Differentialdiagnose von Lyme-Arthritis gegenüber rheumatoider Arthritis, **dadurch gekennzeichnet, dass**:
a) in einer Probe eines Probanden der Gehalt an Myristinsäure enthaltendem Lysophosphatidylethanolamin (LysoPE(14:0), bestimmt wird und
b) der in a) ermittelte Lysophosphatidylethanolamin-Gehalt mit dem Gehalt einer Referenzprobe verglichen wird;
wobei die Referenzprobe eine Probe eines gesunden Kontrollprobanden ist;
wobei die Probe Vollblut, Plasma oder Serum ist; und
wobei ein höherer Gehalt an Myristinsäure enthaltendem Lysophosphatidylethanolamin, als der Gehalt in der Referenzprobe darauf hinweist, dass der Proband an Lyme-Arthritis leidet.

6. Verfahren zur *in*-*vitro*-Differentialdiagnose nach Anspruch 5, **dadurch gekennzeichnet, dass** die Probe Blutplasma ist.

7. Verfahren zur *in*-*vitro*-Differentialdiagnose nach Anspruch 5 oder 6, **dadurch gekennzeichnet, dass** es sich bei dem Probanden um einen Menschen handelt.

8. Verfahren zur *in*-*vitro*-Differentialdiagnose nach einem der Ansprüche 5 bis 7, **dadurch gekennzeichnet, dass** der Gehalt an Myristinsäure enthaltendem Lysophosphatidylethanolamin mittels Flüssigchromatographie-Massenspektrometrie (LC-MS), insbesondere LC-MS/MS, gemessen wird.

9. Verwendung von Myristinsäure enthaltendem Lysophosphatidylethanolamin (LysoPE(14:0)) als Biomarker in der *in-vitro-Diagnose* von Lyme-Arthritis.

10. Verwendung von Myristinsäure enthaltendem Lysophosphatidylethanolamin (LysoPE(14:0)) als Biomarker in der *in*-*vitro*-Differentialdiagnose von Lyme-Arthritis gegenüber rheumatoider Arthritis.

## Revendications

1. Méthode de diagnostic *in vitro* de l'arthrite de Lyme chez un sujet, **caractérisée en ce que** :
a) dans un échantillon prélevé sur un sujet, le taux de lysophosphatidyléthanolamine contenant de l'acide myristique (LysoPE(14:0)) est déterminé, et
b) le taux de lysophosphatidyléthanolamine déterminé en a) est comparé à celui de lysophosphatidyléthanolamine contenant de l'acide myristique présent dans un échantillon de référence ;
l'échantillon de référence étant un échantillon provenant d'un sujet témoin sain ;
l'échantillon étant du sang total, du plasma ou du sérum ; et
si un taux de lysophosphatidyléthanolamine contenant de l'acide myristique est supérieur à celui de l'échantillon de référence, ça indique que le sujet souffre d'arthrite de Lyme.

2. Méthode de diagnostic *in vitro* de l'arthrite de Lyme selon la revendication 1, **caractérisée en ce que** l'échantillon est du plasma sanguin.

3. Méthode de diagnostic *in vitro* de l'arthrite de Lyme selon la revendication 1 ou 2, **caractérisée en ce que** le sujet est un sujet humain.

4. Méthode de diagnostic *in vitro* de l'arthrite de Lyme selon l'une quelconque des revendications 1 à 3, **caractérisée en ce que** le taux de lysophosphatidyléthanolamine contenant de l'acide myristique est déterminé par chromatographie liquide couplée à la spectrométrie de masse (LC-MS), en particulier par LC-MS/MS.

5. Méthode de diagnostic différentiel *in vitro* de l'arthrite de Lyme et de la polyarthrite rhumatoïde, **caractérisée en ce que** :
a) dans un échantillon prélevé sur un sujet, le taux de lysophosphatidyléthanolamine contenant de l'acide myristique (LysoPE(14:0)) est déterminé ; et
b) le taux de lysophosphatidyléthanolamine déterminé en a) est comparé à celui d'un échantillon de référence ;
l'échantillon de référence étant un échantillon provenant d'un sujet témoin sain ;
l'échantillon étant du sang total, du plasma ou du sérum ; et
si un taux de lysophosphatidyléthanolamine contenant de l'acide myristique est supérieur à celui de l'échantillon de référence, ça indique que le sujet souffre d'arthrite de Lyme.

6. Méthode de diagnostic différentiel *in vitro* selon la revendication 5, **caractérisée en ce que** l'échantillon est du plasma sanguin.

7. Méthode de diagnostic différentiel *in vitro* selon la revendication 5 ou 6, **caractérisée en ce que** le sujet est un sujet humain.

8. Méthode de diagnostic différentiel *in vitro* selon l'une quelconque des revendications 5 à 7, **caractérisée en ce que** le taux de lysophosphatidyléthanolamine contenant de l'acide myristique est déterminé par chromatographie liquide couplée à la spectrométrie de masse (LC-MS), en particulier par LC-MS/MS.

9. Utilisation de la lysophosphatidyléthanolamine contenant de l'acide myristique (LysoPE(14:0)) comme biomarqueur dans le diagnostic *in vitro* de l'arthrite de Lyme.

10. Utilisation de la lysophosphatidyléthanolamine contenant de l'acide myristique (LysoPE(14:0)) comme biomarqueur dans le diagnostic différentiel *in vitro* de l'arthrite de Lyme par rapport à la polyarthrite rhumatoïde.
